# EUROPEAN PATENT SPECIFICATION

(11) **EP 2 791 673 B1**
(45) Date of publication and mention of the grant of the patent: **29.08.2018**
(21) Application number: 12858515.5
(22) Date of filing: 14.12.2012
(51) Int. Cl.: G01N 33/48, G01N 33/483, G01N 33/53, G01N 33/68

(54) **LAMIN A/C AND PRELAMINS AS INDICATORS OF FRAILTY AND VULNERABILITY OR RESILIENCY TO ADVERSE HEALTH OUTCOMES**
LAMIN A/C UND PRÄLAMINE ALS INDIKATOREN VON Gebrechlichkeit UND VERWUNDBARKEIT ODER Belastbarkeit FÜR GESUNDHEITSSCHÄDIGENDE ERGEBNISSE
LAMINE A/C ET PRÉLAMINES CONVENANT COMME INDICATEURS DE FRAGILITÉ ET DE VULNÉRABILITÉ OU DE RÉSILIENCE FACE À DES RÉSULTATS CLINIQUES DÉFAVORABLES

(30) Priority: 16.12.2011 US 201161576628 P
(43) Date of publication of application: 22.10.2014
(73) Proprietor: Afilalo, Jonathan, Cote St Luc QC H3X 4B3 (CA); Meyer Ohayon, Samuel, Montreal QC H2J 3N1 (CA); Afilalo, Marc, Cote St Luc QC H4V 1C2 (CA)
(72) Inventor: Afilalo, Jonathan, Cote St Luc QC H3X 4B3 (CA); Meyer Ohayon, Samuel, Montreal QC H2J 3N1 (CA); Afilalo, Marc, Cote St Luc QC H4V 1C2 (CA)
(74) Representative: Cabinet Plasseraud
(86) International application number: PCT/CA2012/050900
(87) International publication number: WO 2013/086637

(56) References cited:
- US-A1- 2010 297 618
- AFILALO J ET AL: "Gait Speed as an Incremental Predictor of Mortality and Major Morbidity in Elderly Patients Undergoing Cardiac Surgery", JOURNAL OF THE AMERICAN COLLEGE OF CARDIOLOGY, ELSEVIER, NEW YORK, NY, US, vol. 56, no. 20, 9 November 2010 (2010-11-09), pages 1668-1676, XP027459594, ISSN: 0735-1097, DOI: 10.1016/J.JACC.2010.06.039 [retrieved on 2010-11-02]
- Jonathan Afilalo: "Frailty Assessment Before Cardiac Surgery Thesis candidate", , 1 January 2011 (2011-01-01), XP055187864, Retrieved from the Internet: URL:http://digitool.library.mcgill.ca/thes isfile92222.pdf [retrieved on 2015-05-06]
- WU ET AL.: 'Reduced expression of lamin A/C correlates with poor histological differentiation and prognosis in primary gastric carcinoma' JOURNAL OF EXPERIMENTAL & CLINICAL CANCER RESEARCH vol. 28, no. 8, 15 January 2009, pages 1 - 12, XP021053022
- MOSS ET AL.: 'Decreased and aberrant nuclear lamin expression in gastrointestinal tract neoplasms' GUT vol. 45, 1999, pages 723 - 729, XP002459319
- TANG ET AL.: 'Promotion of tmnor development in prostate cancer bv progerin' CANCER CELL INTERNATIONAL vol. 10, no. 47, 2010, pages 1 - 10, XP021088181
- TONG ET AL.: 'Lamin A/C deficiencv is associated with fat infiltration of muscle and bone' MECHANISMS OF AGEING AND DEVELOPMENT vol. 132, 01 October 2011, pages 552 - 559, XP055073160
- AFILALO, J.: 'Frailty in Patients with Cardiovascular Disease: Why, When and How to Measure' CURR. CARDIOVASC. RISK. REP. vol. 5, 02 August 2011, pages 467 - 472, XP019947127
- WILLIS ET AL.: 'Lamin A/C Is a Risk Biomarker in Colorectal Cancer' PLOS ONE vol. 3, no. 8, 20 August 2008, page E2988, XP055073161
- LI ET AL.: 'Decreased Bone Formation and Osteopenia in Lamin A/C- Deficient Mice, e19313' PLOS ONE vol. 6, no. 4, 25 April 2011, XP055073162
- AFILALO ET AL.: 'Age-related changes in lamin A/C expression in cardiomyocytes' AMERICAN JOURNAL OF PHYSIOLOGY - HEAT AND CIRCULATORY PHYSIOLOGY vol. 293, 18 May 2007, pages H1451 - H1456, XP055073164
- MALHOTRA ET AL.: 'Lamin A/C deficiency as a cause of familial dilated cardiomyopathy' CURRENT OPINION IN CARDIOLOGY vol. 24, 2009, pages 203 - 208, XP008174631
- HUDSON ET AL.: 'Identification of differentiallv expressed proteins in ovarian cancer using high-density-protein microarravs' PNAS vol. 104, no. 44, 30 October 2007, pages 17494 - 17499, XP055024503

## Description

### FIELD OF THE INVENTION

This application relates to the LMNA gene protein products and their expression associated with the vulnerability to adverse health outcomes observed in some individuals as well as its use to predict the presence or absence of this vulnerability in tested individuals.

### BACKGROUND

Frailty, from the French "frêle" meaning of little resistance, is a syndrome that reflects the state of decreased physiologic reserves and vulnerability to stressors such as illness and/or surgery. Frailty is used interchangeably with the terms "accelerated aging", "unsuccessful aging", and "biological aging" (in contrast to chronological aging). Frailty is at the forefront of the medical agenda, having immediate relevance for clinicians, researchers, policymakers and payers. It is central to the current phenomena of aging, personalized medicine, cost-effectiveness, risk: benefit, and minimally invasive therapeutics. As the number of elderly patients encountered is growing at an alarming rate, frailty is used to gain prognostic insights into this heterogeneous group of patients and thereby tailor treatments on a personalized level which balances risks, benefits, and costs.

AFILALO, J. mentions "Frailty in Patients with Cardiovascular Disease: Why, When and How to Measure", in CURR. CARDIOVASC. RISK. REP., (20110802), vol. 5, pages 467 - 472, XP019947127

Clinical tools are available to measure frailty, these tools include physical performance tests and questionnaires. Clinical frailty assessment tools seek to measure one or more of the seven domains that characterize the clinical phenotype of frailty: slowness, weakness, weight loss, low physical activity, exhaustion, cognitive impairment, and mood disturbance. Epidemiologic studies have shown that frailty confers an increased risk of death and adverse health outcomes. Although more than 20 clinical frailty assessment tools have been developed, there is no consensus agreement on which tool to use. The uncertainty regarding which frailty assessment tool to use is compounded by uncertainty regarding which cutoff to use for each tool, in particular for the physical performance test of gait speed. For example, up to 10 different cutoffs have been proposed for gait speed, ranging from 0.6 m/s to 1.0 m/s in cardiac patients.

Clinical frailty assessment tools have a number of important limitations that restrict their applicability and accuracy. Most of the tools include a test of gait speed (or other test of mobilization) which cannot be performed in non-ambulatory patients, severely ill patients, actively symptomatic patients, or patients restrained or impeded by intravenous lines and catheters. Considering that these patients represent a very high-risk subgroup which could benefit most from frailty data to guide challenging medical decisions, this is a significant problem. These tools suffer from test-retest variability, inter-observer variability, and dependency on patient motivation. They are less accurate in the presence of language barriers and cognitive impairment. They are time-consuming, require equipment or materials, and have had limited success being integrated into the existing clinical workflow

In various cohorts, certain patients are vulnerable to adverse health outcome(s) in response to stressors. Such vulnerability is not easy to predict. For example, certain patients undergoing coronary artery bypass surgery will experience an uneventful recovery, whereas others will experience serious morbidity and mortality. The latter group may derive more harm than benefit from the therapeutic intervention.

It would thus be beneficial to be provided with a non-invasive biomarker linked to the vulnerability (or increased risk) of adverse health outcome. Preferably, this biomarker has the potential to overcome some of the limitations noted above by being objective, reliable, and not dependent on illness acuity, symptoms, cognition, motivation, or physical capacities.

### BRIEF SUMMARY

The present invention is defined by the claims.

The present disclosure provides a method for determining the risk to an adverse health outcome in individuals based on the level of expression of at least one LMNA gene products (e.g. a lamin A/C polypeptide, a lamin precursor polypeptide and/or a ration between the lamin A/C polypeptide and the lamin precursor polypeptide).

In a first aspect, the present disclosure provides a method of determining, in a screened individual, a risk to an adverse health outcome in response to a stressor. Broadly, the method comprises (a) receiving a first test level of expression of at least one LMNA gene product from a biological sample from the screened individual, the biological sample comprising the at least one LMNA gene product; (b)comparing the first test level expression of the at least one LMNA gene product to a first control level of expression of the at least one LMNA gene product to determine if the first test level is lower than, equal to or greater than the first control, wherein the first control level is the level of expression of the at least one LMNA gene product from an age-matched fit control individual; and (c) characterizing the screened individual based on the comparison. The at least one LMNA gene product is selected from the group consisting of a lamin A/C polypeptide, a lamin precursor polypeptide and a ratio of the lamin A/C polypeptide to the lamin precursor polypeptide. When the at least one LMNA gene product is the lamin A/C polypeptide, step (c) further comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. When the at least one LMNA gene product is the lamin precursor polypeptide, step (c) further comprises characterizing the individual as (i) being more susceptible to the adverse health outcome than the control individual if the first test level is greater than the first control level, (ii) being as susceptible to the adverse health outcome as the control individual if the first test level is equal to the first control level or (iii) being less susceptible to the adverse health outcome than the control individual if the first test level is lower than the first control level. When the at least one LMNA gene product is the ratio, step (c) further comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. In an embodiment, the lamin A/C polypeptide is a mature lamin A polypeptide. In another embodiment, the lamin precursor polypeptide is selected from the group consisting of progerin, prelamin A and a combination thereof. In still another embodiment, the determination of the first test level is made by flow cytometry. In yet another embodiment, the screened individual is considered as being frail when the screened individual is characterized as being more susceptible than the control individual. In still another embodiment, the first control level is a range of values of the level of expression of the at least one LMNA gene product obtained from age-matched control fit individuals. In yet another embodiment, the adverse health outcome is at least one of a mortality, a morbidity, a new disease status, a worsening disease status, a complication related to a treatment, an intolerance to a treatment, an impaired quality of life, an impaired functional capacity, an impaired exercise capacity, an impaired cognitive function, an impaired mood, a prolonged recovery and a disability. In still another embodiment, the stressor is at least one of an illness, an injury, a surgery, an interventional procedure, an implantation of a medical device, an administration of a medication, an uptitration of the medication, a chemotherapy, a radiotherapy, an ionizing radiation, a toxic exposure, an environmental exposure, a psychological problem, an oxidative stress, an inflammatory stress, a chronic wear and tear stress and an emotional stress. In an embodiment, the biological sample is from a tissue. In still another embodiment, the tissue is a cardiac tissue such as, for example, an aortic tissue. In another embodiment, the tissue is a lining mucosa, such as an oral mucosa and, in still a further embodiment, a buccal mucosa. In an embodiment, the biological sample comprises epithelial cells, such as, for example, epithelial cells are from a buccal mucosa. In yet a further embodiment, the screened individual is a human.

In a second aspect, the present disclosure provides a kit for determining, in a screened individual, a risk to an adverse health outcome in response to a stressor. The kit comprises
means for detecting at least one LMNA gene product; and a first control level of expression of at least one LMNA gene product from an age-matched fit control individual. The at least one LMNA gene product is selected from the group consisting of a lamin A/C polypeptide, a lamin precursor polypeptide and a ratio of the lamin A/C polypeptide to the lamin precursor polypeptide. In an embodiment, the lamin A/C polypeptide is a mature lamin A polypeptide. In another embodiment, the lamin precursor polypeptide is selected from the group consisting of progerin, prelamin A and a combination thereof. In yet a further embodiment, the kit further comprises means for collecting a cell. In an embodiment, the means for detecting the at least one LMNA gene product comprises a primary antibody or a fragment thereof specific for the at least one LMNA gene product. In a further embodiment, the primary antibody or fragment thereof is labeled. In yet another embodiment, the kit further comprises a secondary labeled antibody specific for the primary antibody. In an embodiment, the kit further comprises at least one of: a container for the collected cell, a solution for storing the cell, a solid support and instructions for collecting the cell. In an embodiment, the cell is from a tissue. In still another embodiment, the tissue is a cardiac tissue such as, for example, an aortic tissue. In another embodiment, the tissue is a lining mucosa, such as an oral mucosa and, in still a further embodiment, a buccal mucosa. In an embodiment, the biological sample comprises epithelial cells, such as, for example, epithelial cells are from a buccal mucosa. In yet a further embodiment, the screened individual is a human. In another embodiment, the means for collecting the cell is a brush. In still another embodiment, the first control level is a range of values of the level of expression of the LMNA gene product provided as a function of age and obtained from aged-matched fit individuals.

In a third aspect, the present disclosure provides a software product embodied on a computer readable medium and comprising instructions for determining a risk to an adverse health outcome in a screened individual in response to a stressor. The product comprises a first receiving module for receiving a first test level of at least one LMNA gene product in a biological sample of the screened individual, wherein the biological sample comprises a nucleus having the at least one LMNA gene product; a first comparison module for determining if the first test level is lower than, equal to or higher than a first control value and generating a corresponding output, wherein the first control value is the level of expression of the at least one LMNA gene product from at least one age-matched fit control individual; and a first characterization module receiving the corresponding output from the first comparison module and adapted to characterize the susceptibility of the screened individual to the adverse health outcome. The at least one LMNA gene product is selected from the group consisting of a lamin A/C polypeptide, a lamin precursor polypeptide and a ratio of the lamin A/C polypeptide to the lamin precursor polypeptide. When the at least one LMNA gene product is the lamin A/C polypeptide, the characterization module comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. When the at least one LMNA gene product is the lamin precursor polypeptide level, the characterization module further comprises characterizing the individual as (i) being more susceptible to the adverse health outcome than the control individual if the first test level is greater than the first control level, (ii) being as susceptible to the adverse health outcome as the control individual if the first test level is equal to the first control level or (iii) being less susceptible to the adverse health outcome than the control individual if the first test level is lower than the first control level. When the at least one LMNA gene product is the ratio, the characterization modules comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. Embodiments of the lamin A/C polypeptide, the lamin precursor polypeptide, the first control level, the adverse health outcome, the stressor, the biological sample and the screened individual are provided herein and do apply to the computer-readable medium.

In a fourth aspect, the present disclosure provides a prognostic system for determining a risk to an adverse health outcome in a screened individual in response to a stressor. The prognostic system comprises a reaction vessel adapted to receive a biological sample from the screened individual and means for detecting at least one LMNA gene product, wherein the biological sample comprises a nucleus of the cell having the at least one LMNA gene product; a processor in a computer system; a memory accessible by the processor; and at least one application coupled to the processor. The at least one application is configured for receiving a first test level of expression of the at least one LMNA gene product from the biological sample; comparing the first level with a first control level to determine if the first test value is lower than, equal to or higher than the first control level, wherein the first control level is the level of expression of the at least one LMNA gene product from at least one age-matched fit control individual; and characterizing the screened individual. When the at least one LMNA gene product is the lamin A/C polypeptide, the application comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. When the at least one LMNA gene product is the lamin precursor polypeptide level, the application comprises characterizing the individual as (i) being more susceptible to the adverse health outcome than the control individual if the first test level is greater than the first control level, (ii) being as susceptible to the adverse health outcome as the control individual if the first test level is equal to the first control level or (iii) being less susceptible to the adverse health outcome than the control individual if the first test level is lower than the first control level. When the at least one LMNA gene product is the ratio, the application comprises characterizing the individual as (i) being more susceptible to the adverse health outcome than the control individual if the first test level of is lower than the first control level, (ii) being as susceptible to the adverse health outcome as the control individual if the first test level is equal to the first control level or (iii) being less susceptible to the adverse health outcome than the control individual if the first test level is higher than the control level. Embodiments of the lamin A/C polypeptide, the lamin precursor polypeptide, the first control level, the adverse health outcome, the stressor, the biological sample and the screened individual are provided herein and do apply to the diagnostic system.

### BRIEF DESCRIPTION OF THE DRAWINGS

Having thus generally described the nature of the invention and of the disclosure, reference will now be made to the accompanying drawings, showing by way of illustration, a preferred embodiment thereof, and in which:

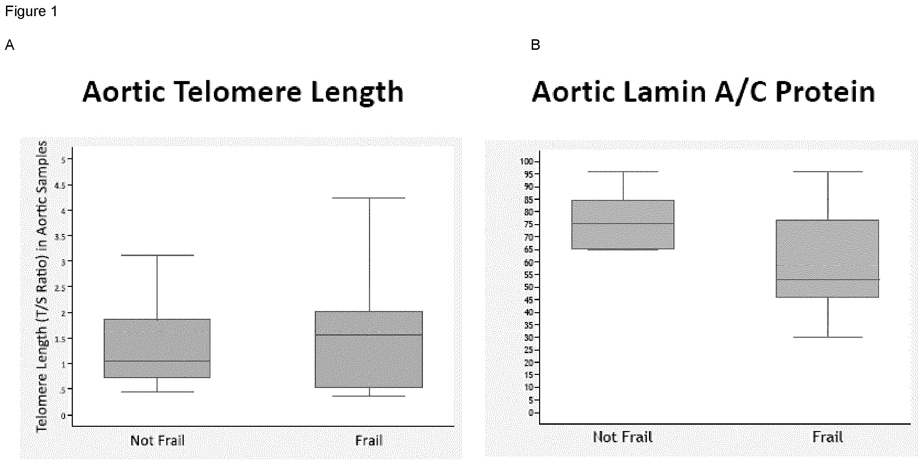

**Figure 1** illustrates the telomere length and the lamin A/C expression in aortic samples in frail and non-frail individuals. **A** The telomere length (telomere-to-single copy gene or T/S ratio) is provided as a function of patient's type (not frail vs. frail). **B** The lamin A/C protein expression (% of nuclei expressing lamin A/C protein above the positive threshold) is provided as a function of patient's type (not frail vs. frail).

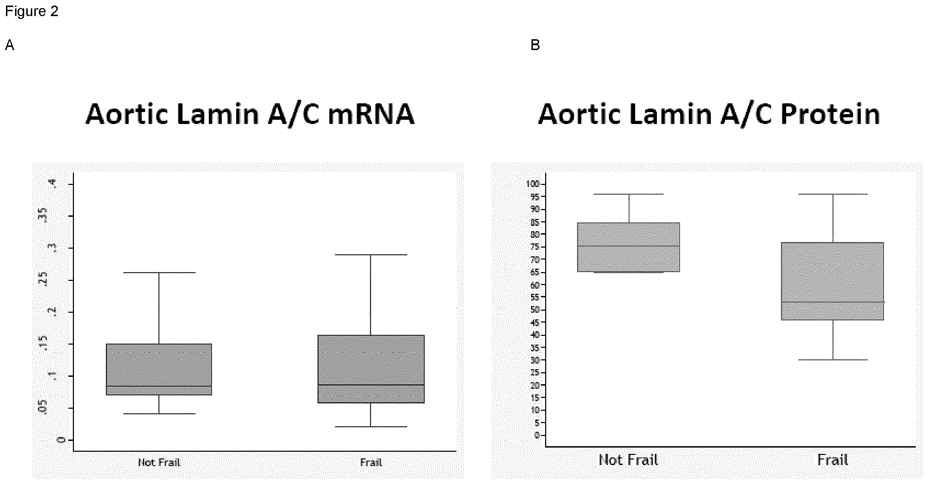

**Figure 2** illustrates the lamin A/C mRNA and protein expression in aortic samples of frail and non-frail individuals. **A** The lamin A/C mRNA expression (normalized to GDPH expression) is provided as a function of patient's type (not frail vs. frail). B The lamin A/C protein expression (% of nuclei expressing lamin A/C protein above the positive threshold) is provided as a function of patient's type (not frail vs. frail).

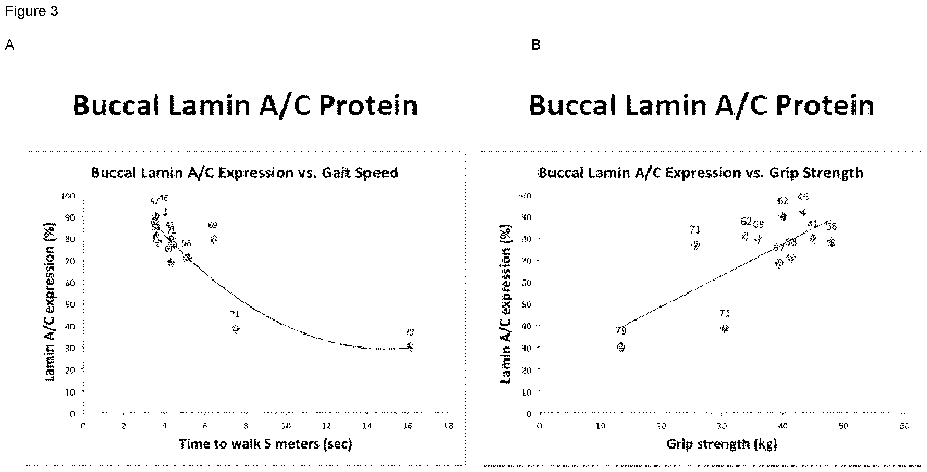

**Figure 3** illustrates the lamin A/C protein expression in cells from buccal swab. **A** The lamin A/C protein expression (% of nuclei expressing lamin A/C protein above the positive threshold) is provided as a function of gait speed (*e.g.* the time to walk five meters (seconds). **B** The lamin A/C protein expression (% of nuclei expressing lamin A/C protein above the positive threshold) is provided as a function of grip strength (kg).

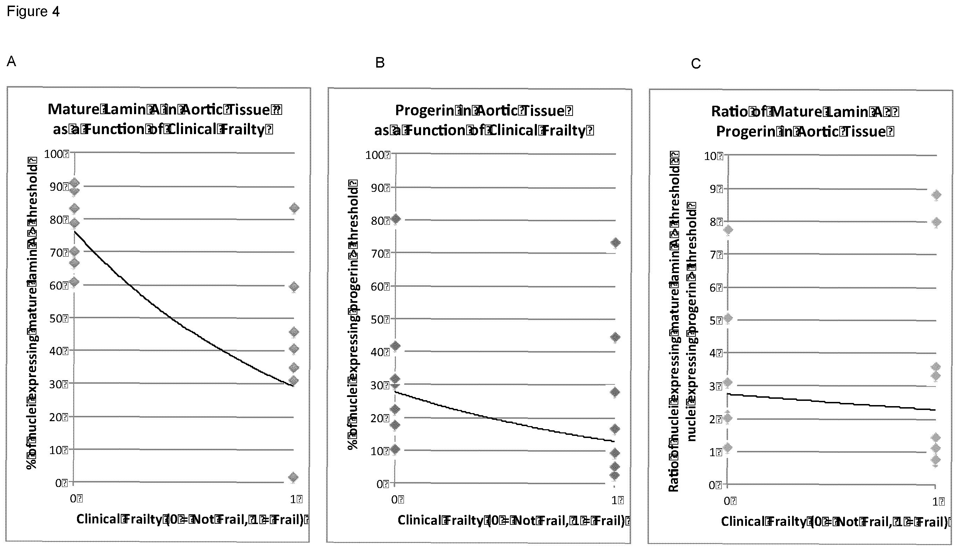

**Figure 4** illustrates the mature lamin A, progerin, and their ratio as a function of the clinical frailty status (determined according to Fried's scale) in aortic tissues as measured by flow cytometry. **A** The mature lamin protein expression (% of nuclei expressing lamin A/C protein above the positive threshold) is provided as a function of frailty status (0=not-frail, 1= frail). **B** The progerin protein expression (% of nuclei expressing progerin protein above the positive threshold) is provided as a function of frailty status (0=not-frail, 1= frail). **C** The ration of mature lamin expression (% of nuclei expressing lamin A/C protein above the positive threshold) to progerin expression (% of nuclei expressing progerin protein above the positive threshold) is provided as a function of frailty status (0=not-frail, 1= frail).

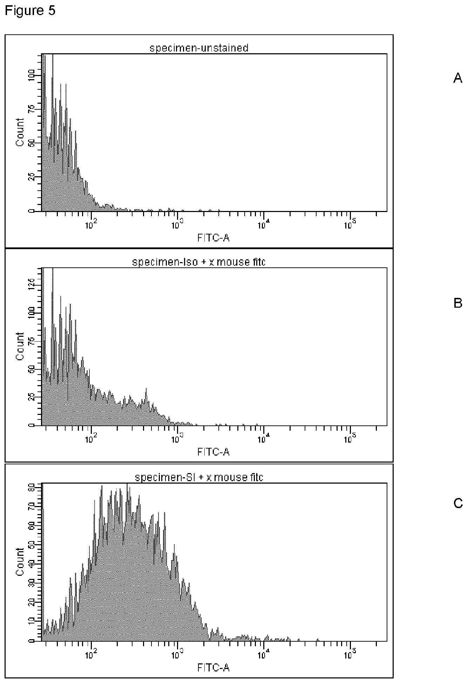

**Figure 5** provides flow cytometry histograms showing the fluometric counts as a function of FITC fluorescence of unstained buccal cells **(A),** buccal cells treated with the control isotype **(B)** or buccal cells treated with anti-lamin A/C antibody with corresponding FITC-labeled secondary antibody **(C).**

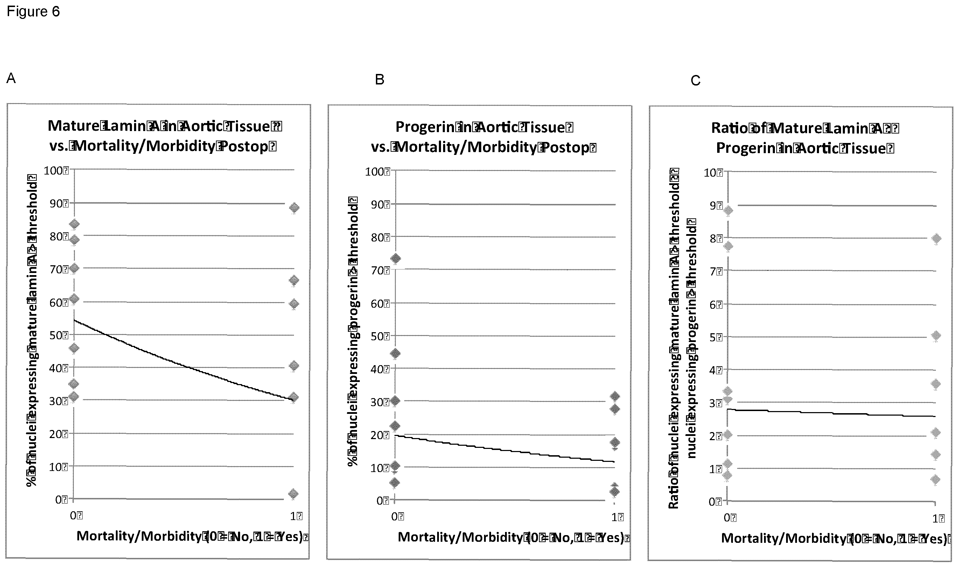

**Figure 6** illustrates the mature lamin A, progerin, and their ratio as a function of post-operative mortality and major comorbidity in aortic tissues. **A** The mature lamin protein expression (% of nuclei expressing lamin A/C protein above the positive threshold) is provided as a function of incidence of mortality and major comorbidity (0=no incidence, 1= incidence). **B** The progerin protein expression (% of nuclei expressing progerin protein above the positive threshold) is provided as a function of incidence of mortality and major comorbidity (0=no incidence, 1=incidence). **C** The ration of mature lamin expression (% of nuclei expressing lamin A/C protein above the positive threshold) to progerin expression (% of nuclei expressing progerin protein above the positive threshold) is provided as a function of incidence of mortality and major comorbidity (0=no incidence, 1=incidence).

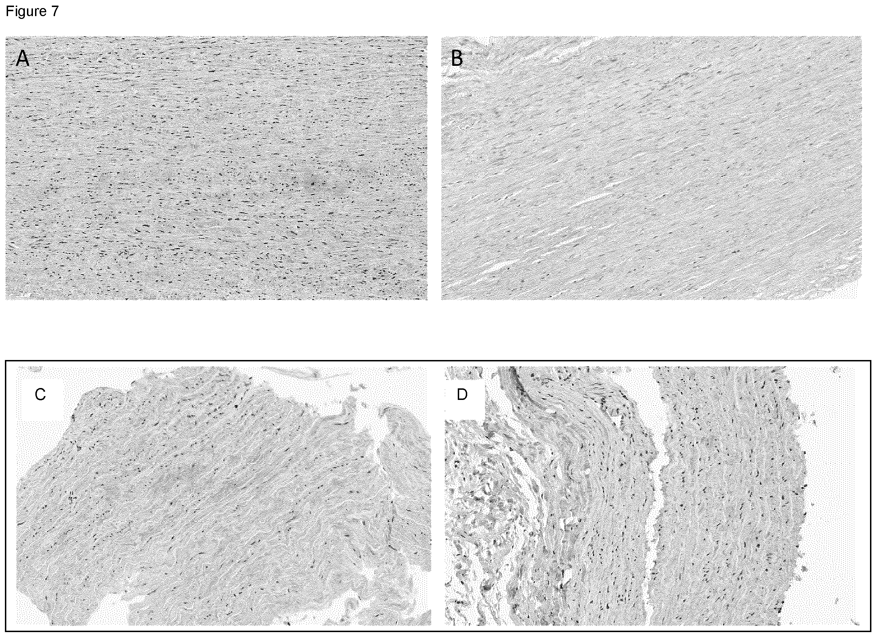

**Figure 7** provides an immunohistolochemistry of formalin-fixed paraffin-embedded aortic punch sections using an anti-mature lamine A antibody (panels **A** and **B**) as well as with an anti-progerin antibody (panels **C** and **D**). The results obtained from a 46 year-old fit patient (panel A) and from a 77 year-old frail patient (panel B) indicate that the fit patient expresses a higher level of the mature lamin A polypeptide than the frail one. The results obtained from a 70 year-old fit patient(C)and from a 86 year-old frail patient (D) indicate that the fit patient expresses a lower level of progerin than the frail one.

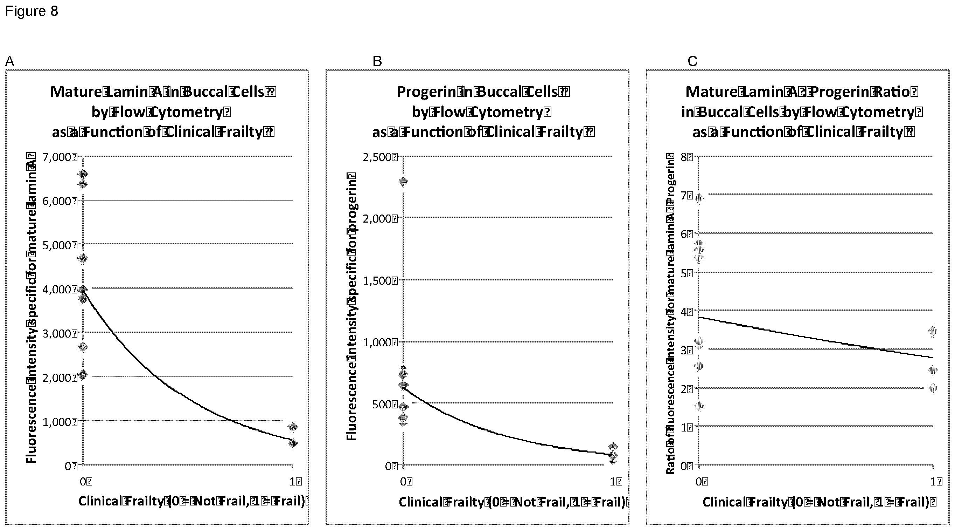

**Figure 8** illustrates the mature lamin A, progerin, and their ratio as a function of the clinical frailty status (determined according to Fried's scale) in buccal cells as measured by flow cytometry. **A** The mature lamin protein expression (% of nuclei expressing lamin A/C protein above the positive threshold) is provided as a function of frailty status (0=not-frail, 1= frail). **B** The progerin protein expression (% of nuclei expressing progerin protein above the positive threshold) is provided as a function of frailty status (0=not-frail, 1= frail). **C** The ration of mature lamin expression (% of nuclei expressing lamin A/C protein above the positive threshold) to progerin expression (% of nuclei expressing progerin protein above the positive threshold) is provided as a function of frailty status (0=not-frail, 1= frail).

### DETAILED DESCRIPTION

The present invention is based on a modulation in the expression of the LMNA gene product in individuals predisposed to an increased risk of an adverse health outcome in response to a stressor.

### Definitions

Throughout this application, various terms are used according to their plain meaning in the art. However, some of them are more precisely defined herein.

*Adverse health outcome in response to a stressor.* The methods and associated products described herein are designed to assess the risk to an adverse health outcome in response to a stressor. A "stressor" refers to a stimulus which imparts physiological stress, and may broadly include illness, injury, infection and/or iatrogenic stressors. The term "illness" encompasses acute and chronic diseases (such as but not limited to cardiovascular disease, infectious disease, autoimmune disease, and cancer) as well as periods of sickness. The term "injury" encompasses traumatic or non-traumatic injuries, and may include athletic injuries. Physical therapy, exercise training, smoking, sun exposure, environmental exposures, ionizing radiation exposure, and mental stressor also represent examples of potential stressors. The term "Iatrogenic stressors" encompasses surgery, interventional procedures, implantation of medical devices, administration or up-titration of medications, chemotherapy, radiotherapy, etc. In a clinical setting, it is often necessary to apply an iatrogenic stressor to a patient to prevent, treat or alleviate a symptom associated with a particular illness or injury. Depending on the desired result, various treatment options can be envisaged. However, it is sometimes difficult to determine if a more aggressive/invasive treatment would provide additional benefit to the patient without adding an additional health burden because very few methods can predict the outcome of the application of a stressor.

As used herein, an "adverse health outcome" refers to a negative (or bad) health condition or status that can arise when a stressor is applied. An adverse health outcome decreases the patient's health status and can even lead to death. In an embodiment, the adverse health outcome in response to a stressor identifies patients suffering from the frailty syndrome. In an embodiment, the adverse health outcome can be, for example, mortality, morbidity (including development of new comorbid illness or worsening of known illnesses), complications related to a treatment (including but not limited to postoperative complications), intolerance to a treatment (including but not limited to adverse drug events), impaired quality of life, impaired functional capacity, impaired exercise capacity, impaired cognitive function or mood, increased healthcare resource utilization, increased health care cost, prolonged length of stay in hospital, hospital admissions, institutionalization and disability. In some embodiments, the adverse health outcome can be, for example, increased healthcare cost, an hospital admissions, an increased healthcare resource utilization, a prolonged length of stay in an hospital, an increased health care cost, institutionalization, a need for rehabilitation, a need for support services.

*Biological sample.* A biological sample is a sample of an individual's tissues and/or cells collected from the tissue. In this present application, the biological sample can be derived from any tissue. In an embodiment, the biological sample can be from a cardiac tissue (such as an aorta). In another embodiment, the biological sample can be from a mucosa, such as, for example, a lining mucosa (e.g. the buccal mucosa). In a preferred embodiment, the biological sample comprises epithelial cells collected from the tissue (e.g. epithelial cells from a mucosa, for example, a lining mucosa, such as the buccal mucosa). In another embodiment, the biological sample essentially comprises epithelial cells (at least 95% of the collected cells). In another embodiment, the biological sample comprises fibroblasts, muscle cells (such as smooth muscle cells and pericytes) and/or endothelial cells. In still another embodiment, the biological sample is substantially devoid of blood-derived cells (such as red blood cells, lymphocytes, etc.). In an embodiment, the biological sample is not substantially derived from biological fluid (even though it may contain some biological fluids).

Since the lamin A/C biomarker, upon protein processing, is located within the nucleus (e.g. nuclear lamina and nuclear matrix), the biological sample comprises at least one nucleus. The nucleus can be present within a cell obtained from the tissue, can be present in an extract of the cell or can be substantially isolated from the cell.

Since the lamin precursors (or prelamins, or immature lamins) biomarker, upon protein processing, are located within the nucleus (*e.g*. the nuclearplasm), the biological sample (in embodiments where the lamin precursors polypeptide is detected and quantified) comprises at least one nucleus. The nucleus can be present within a cell obtained from the tissue, can be present in an extract of the cell or can be substantially isolated from the cell.

In an embodiment, the biological sample is obtained by scrapping the buccal mucosa of an individual. In such embodiment, the raw (e.g. untreated) biological sample comprises at least 100 000 cells/mL, at least 300 000 cells/mL or at least 600 000 cells/mL.

The biological sample can be used without prior modification in the various methods described herein. Optionally, the biological sample can be treated (mechanically, enzymatically, etc.) prior to the assays described herein.

*Frailty.* As used herein, "frailty" or the "frailty syndrome" is a geriatric syndrome that reflects the state of decreased physiologic reserves and vulnerability to stressors related to accelerate aging. The pathophysiology of frailty is rooted in dysregulation of multiple interrelated systems, particularly the immune, hormonal, and endocrine systems. Frail individuals share a number of clinical attributes which have been measured and operationalized into clinical frailty scales. These attributes include, but are not limited to, unintentional weight loss, muscle weakness, slow walking speed, exhaustion, low physical activity, cognitive impairment, and mood disturbance. As indicated above, a measure of frailty can be obtained clinically. By contrast, an individual who is not considered frail is a "fit" individual.

*LMNA gene products.* As used herein, the term "LMNA" gene products refers to polypeptides encoded by the LMNA gene. It includes mature lamins, such as lamin A and lamin C (sometimes referred to as lamin A/C) as well as lamin precursors, such as progerin and prelamin A.

*Reaction vessel.* The reaction vessel is a discrete unit where a biological sample is placed. The reaction vessel is an *in vitro* environment. In an embodiment, it contains cells, cellular extracts and/or subcellular components (such as the nucleus) obtained from the biological sample. The reaction vessel can include, for example, means for detecting the lamin A/C polypeptide and/or the lamin precursor polypeptide as well as any reagents necessary to perform the detection and semi-quantification (or quantification) of the level of expression of the polypeptide markers.

### Lamin A/C and laminopathies

Lamin A and C (A/C) (also referred to as the mature LMNA polypeptides) are type V intermediate filaments encoded by the LMNA gene that form the nuclear lamina. The functions of lamin A/C are to support the inner nuclear envelope and to participate in DNA repair, signal transduction, mesenchymal stem cell differentiation, mitosis, and apoptosis.

Lamin A/C is generated from the post-transcriptional modification and proteolytic cleavage of lamin precursors (such as, for example, prelamin A). In some individuals who are more vulnerable to an adverse health outcome in response to a stressor, the level of the lamin A/C polypeptide expression is reduced with respect to the level of the lamin A/C polypeptide expression of an age-matched fit individual. As shown herein, this reduction in expression is observed only at the polypeptide level and not at the mRNA level. As also shown herein, this reduction in expression is not caused by a genetic anomaly in the LMNA gene which is associated progeria and known laminopathies. As further shown herein, this reduction in expression is not observed in bodily fluids (such as blood or urine), but is measured in bodily tissues or cells from bodily tissues.

In some individuals who are more vulnerable to an adverse health outcome, the level of expression of lamin precursors is increased with respect to the level of expression of lamin precursors of an age-matched fit individual. As used herein, the term "lamin precursor(s)" refer to all polypeptides produced from the expression of the LMNA gene (whose genetic sequence is not associated with a known laminopathy), excluding the mature form of lamin A/C. Lamin precursors include, but are not limited to, prelamin A and progerin. Progerin can be detected in individuals not afflicted by a genetically-caused laminopathy as a results of differential splicing. Even though the post-transcriptional modification of progerin cannot lead to the formation of a mature lamin A/C polypeptide, in the context of this application, progerin is referred to as a lamin precursor. As shown herein, this increase in expression is observed only at the polypeptide level and not at the mRNA level. As also shown herein, this increase in expression is not caused by a genetic anomaly of the LMNA gene which is associated with progeria as well as known laminopathies. As further shown herein, this increase in expression is not observed in bodily fluids (such as blood or urine), but is measured in bodily tissues or cells from bodily tissues.

In some individuals who are more vulnerable to an adverse health outcome in response to a stressor, the ratio between the level of the lamin A/C polypeptide expression and the level of lamin precursors polypeptides is reduced with respect to the ratio of an age-matched fit individual. As shown herein, this reduction in expression is observed only at the polypeptide level and not at the mRNA level. As also shown herein, this reduction in expression is not caused by a genetic anomaly in the LMNA gene which is associated progeria and known laminopathies. As further shown herein, this reduction in expression is not observed in bodily fluids (such as blood or urine), but is measured in bodily tissues or cells from bodily tissues.

The Accession Number for human lamin A/C is AAH00511, for human progerin is AAR29466, for human prelamin A/C isoform 1 precursor is NP_733821, for human prelamin A/C isoform 2 is NP_005563 and for human prelamin A/C isoform 3 is NP_733822. The Gene ID NO for the LMNA gene is 4000.

The methods, commercial packages, software products and systems provided herewith are useful to assess the risk in individuals expressing a lamin A/C polypeptide and lamin precursors encoded by a gene which is not associated with known laminopathies. The lamin A/C polypeptide and its precursors can include conservative amino acid modifications as well as amino acid additions or deletions. As such, the methods and associated products provided herewith are useful to assess a decrease in expression in lamin A/C in individuals who, previously in life, did express a physiological level of lamin A/C.

As indicated above, the methods described herein cannot be applied to individuals afflicted by a deleterious mutation in the LMNA gene and afflicted by a known laminopathy. LMNA mutations (associated with "laminopathies") lead to a profound and premature reduction of lamin A/C expression and a toxic accumulation of its precursors leading to diverse phenotypes characterized by accelerated aging. These clinical phenotypes include a number of genetic diseases predominantly affecting the cardiac and neuromuscular systems: progeria (Hutchison-Gilford syndrome), Emery-Dreifuss muscular dystrophy, limb-girdle muscular dystrophy, Charcot-Marie-Tooth type 2 neuropathy, partial lipodystrophy, and familial cardiomyopathy.

Progeria is the classical disease associated with lamin A/C and prelamins. It is a very rare disease in which there is markedly premature aging of multiple organ systems and death in childhood or adolescence. Progeria is caused by a single-point mutation resulting in a silent change at exon 11 of the LMNA gene. This mutation activates a cryptic splice site resulting in the in-frame deletion of 50 amino acids within C-terminus of the LMNA protein. This abnormal prelamin A called progerin or LAΔ50 still retains its CAAX motif but lacks the ZMPSTE/FACE1 cleavage site which results in its permanent farnesylation and carboxymethylation, and ultimately in its abnormal association into the nuclear lamina throughout the cell cycle. Lamin-related familial cardiomyopathy is more common, affecting up to 30 000 individuals in North America, but grossly underdiagnosed due to the lack of a readily available test to assess lamin A/C expression.

As indicated above, the methods and associated products described herein do not apply to individuals affected by progeria or related laminopathies (progeroid syndromes). The methods and associated products are useful in individuals who express a lamin A/C polypeptide and who, previously in life, expressed a physiological level of lamin A/C.

### Predictive methods and associated software product and systems

The diagnostic and prognostic methods described herein are designed to capture the relationship between the level of LMNA gene product polypeptide expression and the risk of adverse health outcome in response to a stressor in order to generate valuable information about the individual that is being tested. Once an individual has been diagnosed by one of the methods described herein, this individual can be treated according to the therapeutic regimen that is considered appropriate depending on the patient's characterized disease status.

The present application provides methods and associated products in which cells (or cellular extracts) derived from a tissue are analyzed. It is provided that any bodily tissue can be used in the present method. In an embodiment, the tissue may be a cardiac tissue, such as, for example, an aortic tissue. Another tissue which is particularly useful in the present method is a mucosa, such as the oral mucosa and more specifically, the buccal mucosa. Such tissue can easily be obtained non-invasively using, for example, a buccal swab. The biological sample obtained from the oral mucosa includes mucosal epithelial cells. In an embodiment, it can also include fibroblasts, smooth muscle cells, adipocytes and preadipocytes, endothelial cells, etc. As it will be shown below, cells (as well as nuclei associated thereto) from the buccal mucosa were successfully used for LMNA-encoded protein product quantification.

In predictive applications, the biological sample of an individual can then be placed in a reaction vessel. The biological sample comprises the at least one LMNA gene product. In some embodiments, the biological sample comprises a nucleus having a lamin A/C polypeptide. In some embodiment, the biological sample can also comprise a nucleus having a lamin precursor polypeptide. In other embodiments, the biological sample can comprise a cell. In still another embodiment, the nuclear lamina and nuclear matrix, the nucleus or the cell of the biological sample can be derived from a tissue and is not derived from a bodily fluid. In the assays, the reaction vessel can be any type of container that can accommodate the determination of the expression level of the LMNA gene product, in some embodiment, the level of expression of the lamin precursor polypeptide.

Once the biological sample has been placed in the reaction vessel, the expression level of the LMNA gene product is determined. This determination may be made directly in the reaction vessel (by using a probe) or on a sample of such reaction vessel.

This determination step can rely on the addition of a qualifier specific to the polypeptide to be quantified. The qualifier can, for example, specifically bind to a sequence, a subsequence or an epitope of the LMNA gene product. In an embodiment, this binding between the qualifier and the LMNA gene product may be specific to the LMNA gene product (e.g. the qualifier only specifically binds to the lamin A/C and not to one of its precursors such as prelamin A). The association between the qualifier and its target polypeptide can be used to provide the level of expression of the polypeptide. In one embodiment, the qualifier can be an antibody or an antigen-binding fragment thereof capable of specifically recognizing the polypeptide. In instances where the level of expression of the lamin precursor polypeptide is determined, a qualifier can also be used to bind to a sequence, a subsequence or an epitope of the lamin precursor polypeptide. In an embodiment, this binding between the qualifier and the lamin precursor polypeptide may be specific to the lamin precursor polypeptide (*e.g*. the qualifier only specifically binds to the lamin precursor and not to one of its mature form lamin A/C).

It is possible to use the same qualifier to quantify the expression of lamin A/C and its precursor. For example, since the lamin precursors are present in the nucleus and generally not in the nuclear envelope or matrix, it is possible to use a single qualifier for detecting lamin A/C and its precursor. If it is necessary to obtain values for each polypeptide individually, the cellular contents (nuclear envelope, nuclear matrix and nucleoplasm) can be first separated and then each fraction quantified with the single qualifier.

However, it is also possible to use at least two distinct qualifiers to determine the level of expression of lamin A/C and its precursors. In this embodiment, each qualifier would specifically bind to one polypeptide and not the other. As such, the quantification of each qualifier individually (for example by using distinct labels) would allow for the determination of a protein expression level specific for each polypeptide.

If a qualifier is used to determine the level of expression of the lamin precursors and/or the lamin A/C polypeptide, then various techniques known in the art are available to make a semi-quantitative or quantitative measure. Such techniques include, but are not limited to, Western Blot, immuno-fluorescence, immuno-histochemistry, flow cytometry, radioimmuno assay (RIA), enzyme-linked immunosorbent assay (ELISA), protein micro-array, lectin-based assays, etc.

One particular useful method for performing the polypeptide detection is flow cytometry. As will be shown herein, the detection of the lamin A/C polypeptide and the lamin precursor in the nucleus provide valuable information on the risk of an individual to an adverse health outcome in response to a stressor. Flow cytometry enables the detection of the lamin precursors and/or lamin A/C polypeptides at either the nucleus or the nuclear envelope because the cellular structure (even though altered by the flow cytometry processing) remains present. Flow cytometry is a rapid, quantitative, reproducible, method and is amenable to high-throughput screening. In addition, flow cytometry does not require a substantial amount of manual processing has high precision and low inter/intra-observer reliability.

However, the presence of a qualifier is not necessary to provide for the quantification of the lamin A/C and/or lamin precursor polypeptide. Techniques are known in the art for the specific quantification of polypeptides which do not require the presence of a specific qualifier. Those techniques include, but are not limited to mass spectrometry and capillary electrophoresis. In order to use such techniques, it may be necessary to first partially or substantially isolate the nuclear envelope, nuclear matrix, nucleoplasm and/or the nucleus from the collected cells of the biological sample and then submit such cellular extracts to mass spectrometry or capillary electrophoresis.

As also known in the art, the lamin A/C polypeptide interacts with other polypeptides such as, for example, LEM domain protein (such as, but not limited to, Emerin, LAP2, MAN1 and BAF), SUN domain protein, CTCF, pRB, PP2A, MLIP, Lamin B1, Rb, Lco1 and prelamin A. It is also known that the mature lamin polypeptide can form homodimers as well as heterodimers. As such, an assessment of lamin A/C's level of expression can also include the determination of the presence of one (or more) of its binding partners. Alternatively, an assessment of lamin A/C's level of expression can also use one (or more) of its binding partners to as a qualifier.

Alternatively or optionally, an assessment of lamin A/C's biological activity or its precursors can be used as an indirect indicator of the level of expression the polypeptides. As indicated above, lamin A/C is an intermediate filament protein of the nuclear envelope. A measure of the integrity of the nuclear envelope can be used as an indirect indicator of lamin A/C's level of expression.

Once the level of expression has been determined, the information is extracted from the reaction vessel and is compared to at least one control value. In predictive applications, it must be determined if the level of expression of the LMNA gene product obtained from the biological sample is lower than, equal to or higher than a control value. The control value is derived from at least one fit individual who is age-matched with the individual who is being screened. As used herewith, the term "fit" refers to an individual who does not present comorbidity, disability and/or clinical fraily markers. "Fit" is used interchangeably with "normal aging" and "successful aging". As used herein, the term "age-matched" refers to control individuals who have a substantially similar chronological age (in years) than individuals who are screened. In an embodiment, the age-matched control individuals may be the same chronological age as the screened individual. In another embodiment, the age-matched control individuals may have up to (and no more than) a one-year difference in chronological age with the screened individual. In another embodiment, the age-matched control individuals may have up to (and no more than) a two-year difference in chronological age with the screened individual. In still another embodiment, the age-matched control individuals may have up to (and no more than) a five-year difference in chronological age with the screened individual. In yet another embodiment, the age-matched control individuals may have up to (and no more than) a seven-year difference in chronological ages with the screened individual. In still yet another embodiment, the age-matched control individuals may have up to (and no more than) a ten-year difference in chronological ages with the screened individual. In still yet another embodiment, the age-matched control individuals may have up to (and no more than) a thirty-year difference in chronological ages with the screened individual.

In an embodiment, the control level is derived from at least one control fit individual. In yet another embodiment, the control level is a range of values derived from a plurality of control fit individuals. In still another embodiment, the control value is a predetermined value derived from a cohort of control fit individuals. In still another embodiment, the control value is a range of values (also referred to as a datastructure) of the level of expression of the LMNA gene product provided as a function of age and obtained from a plurality of control fit individuals.

In an embodiment, the control value is a compilation of values of levels of expression of the LMNA gene products in control individuals. This compilation can be embodiments in a normogram which would provide the assessed risk, the degree of frailty and/or the biological age based on the levels of expression of the LMNA gene product. The normogram can be derived from the measurements of the LMNA gene product in age-stratified fit volunteers (e.g. presenting no significant comorbidity, no significant cognitive impairment, no disability and/or no clinical frailty markers). In some embodiment, a sex stratification can be made to derive the expected control expression for males and females or for males and females. In still a further embodiment, the normogram can be provided for each decade of life.

In an embodiment, the comparison (between the test level of the individual that is being screened and the control value) can be made by an individual. In another embodiment, the comparison can be made in a comparison module. Such comparison module may comprise a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to determine if the test level of lamin A/C protein expression is lower than the control value. An output of this comparison may be transmitted to a display device. The memory, accessible by the processor, receives and stores data, such as the control level of expression (*e.g*. the normogram) or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

Once the comparison between the test level of expression of the screened individual and the control level of expression of the control individual has been made, then it is possible to characterize the screened individual. This characterization is possible because, as shown herein, a modulation in the expression level of the LMNA gene product is associated with an increased risk (susceptibility or vulnerability) to an adverse health outcome if (or when) a stressor was to be applied to the screened individual.

If it is observed that the level of lamin A/C polypeptide expression is reduced with respect to the control level, that the level of lamin precursor polypeptide expression is increased with respect to the control level and/or that the ratio between the level of lamin A/C polypeptide expression and the level of lamin precursor polypeptide expression is decreased when compared to a control ratio, the individual is characterized as being at risk (vulnerable or susceptible) to an adverse health outcome when compared to a control fit individual. In some embodiments, it is possible to characterize the individual as either being frail or even pre-fail depending if the test level for LMNA gene product or the ratio is slightly (e.g. pre-frail) or substantially (e.g. frail) lower than the control level or if the test level for the lamin precursor is slightly (e.g. pre-frail) or substantially higher (e.g. frail) than the control level.

If it is observed that the level of lamin A/C polypeptide expression is similar to the control level, that the level of lamin precursor polypeptide expression is similar to the control level and/or that the ratio between the level of lamin A/C polypeptide expression and the level of lamin precursor polypeptide expression is similar to a control ratio, then the individual is characterized as not presenting an increased risk to an adverse health outcome when compared to a control fit individual. In such instances, the screened individual is considered fit.

If it is observed that the level of lamin A/C polypeptide expression is increased with respect to the control level, that the level of lamin precursor polypeptide expression is decreased with respect to the control level and/or that the ratio between the level of lamin A/C polypeptide expression and the level of lamin precursor polypeptide expression is increased when compared to a control ratio, the individual is characterized as being at lower risk (e.g. resilient, robust or very fit) to an adverse health outcome when compared to a control fit individual.

In an embodiment, the screened individual's characterization can be made by an individual. In another embodiment, the characterization can be made with a processor and a memory card to perform an application. The processor may access the memory to retrieve data. The processor may be any device that can perform operations on data. Examples are a central processing unit (CPU), a front-end processor, a microprocessor, a graphics processing unit (PPU/VPU), a physics processing unit (PPU), a digital signal processor and a network processor. The application is coupled to the processor and configured to characterize the individual being tested. An output of this characterization may be transmitted to a display device. The memory, accessible by the processor, receives and stores data or any other information generated or used. The memory may be a main memory (such as a high speed Random Access Memory or RAM) or an auxiliary storage unit (such as a hard disk, a floppy disk or a magnetic tape drive). The memory may be any other type of memory (such as a Read-Only Memory or ROM) or optical storage media (such as a videodisc or a compact disc).

The characterization results can either be obtained directly or can be provided remotely. For example, in one embodiment, a user provides the LMNA gene product level to a laboratory, which in return, performs the comparison and the characterization and provides the results to the user, either on a tangible medium or electronically.

The present disclosure also provides diagnostic and prognostic systems for performing the characterizations and methods described herein. These systems comprise a reaction vessel for placing the biological sample, a processor in a computer system, a memory accessible by the processor and an application coupled to the processor. The application or group of applications is(are) configured for receiving a test value for the test level of expression of the LMNA gene product; comparing the test level with a control level and/or characterizing the individual as a function of this comparison.

The present disclosure also provides a software product embodied on a computer readable medium. This software product comprises instructions for characterizing the individual according to the methods described herein. The software product comprises a receiving module for receiving a test level of expression of the LMNA gene product from a biological sample; a comparison module receiving input from the measuring module for determining if the test level is lower than the control level; a characterization module receiving input from the comparison module for performing the characterization based on the comparison.

In an embodiment of the disclosure, an application found in the computer system of the system is used in the comparison module. A measuring module extracts/receives information from the reaction vessel with respect to the level of expression of the LMNA gene product. The receiving module is coupled to a comparison module which receives the value(s) of the test level and determines if this value is lower than the control value. The comparison module can be coupled to a characterization module.

In another embodiment of the disclosure, an application found in the computer system of the system is used in the characterization module. The comparison module is coupled to the characterization module which receives the comparison and performs the characterization based on this comparison.

In a further embodiment of the disclosure, the receiving module, comparison module and characterization module are organized into a single discrete system. In another embodiment, each module is organized into different discrete system. In still a further embodiment, at least two modules are organized into a single discrete system.

The methods and associated products described herein are useful for determining the risk to an adverse health outcome if a stressor is applied to an individual. This information may be used to tailor a medical regimen that is appropriate for the risk of this individual to an adverse health outcome. The medical regimen that would be considered more appropriate can include the use of less invasive interventions when it is determined that the individual is more at risk to an adverse health outcome than a control fit individual

For example, the method described herein can help to predict a patient's response or resiliency to the stressor of a surgery. If it is determined that the screened individual more susceptible to an adverse heath outcome upon surgery, it may be recommended to seek an alternative solution, such as minimally invasive or even non-invasive therapeutics. In another example, the method described herein can help to predict a patient's response or resiliency to the stressor of a chemotherapy. If it is determined that the screened individual more susceptible to an adverse heath outcome upon the administration of a chemotherapy, it may be recommended to seek an alternative solution, such as a less aggressive dosage regimen.

The methods described herein can further be used to stratify cohorts of patients according to their respective risk to the adverse health outcome. Such stratification may be useful in clinical trials to measure the efficacy of a medical device or a therapeutic regimen in frail (or non-frail) patients. The stratification process can also be used to determine if the adverse health events that were observed during the clinical trial are associated (or not) with the presence of the frailty syndrome.

The methods described herein can also be used to characterize the screened individual as being afflicted (*e.g.* frail) or susceptible of being afflicted (*e.g.* pre-fail) with a frailty syndrome. As indicated above, the frailty syndrome or pre-frail condition is not associated with a laminopathy caused by a genetic alteration in the LMNA gene. As shown herein, frail patients show a modulation in LMNA gene products when compared to their non-frail counterparts.

Consequently, the method can be used to diagnose frailty or a pre-frail condition in the screened individuals.

The methods described herein can further be used to evaluate the same individual over the course of a time period. Such evaluation can be useful to determine if a therapy that was applied was useful to increase the resiliency to stressors and/or to improve the biological age of the screened individual. For example, an individual may receive a therapy (for example a drug therapy) to prevent, treat and/or alleviate the symptoms associated to a specific condition. In such example, it may be advisable to determine the level of LMNA gene product before the therapy is undertaken and after the therapy is undertaken. To determine if the therapy is useful in increasing the resiliency in an individual or improving its biological age, the method further comprises comparing the level of expression (or the ratio between the levels of expression) to determine if the therapy is useful. A relative modulation in the level of LMNA gene product expression from the time before the therapy was initiated to a time after the therapy was undertaken would indicate that the therapy is useful in increasing resiliency and or improving the individual's biological age. Such information can also be used to determine if a therapy should be continued or stopped based on its usefulness.

The methods described herein can also be combined with other known techniques to determine an individual's biological age or "true age". For example, in an embodiment, the method described herein can be combined with a clinical evaluation and/or questionnaire.

### Commercial packages

The methods described herein can be performed using a commercial package or kit. The kit comprises at least two components: means for detecting a LMNA gene product as well as a control value of the a level of LMNA gene product expression from at least one age-matched fit individual. In an embodiment, the kit can also comprise a level of lamin A/C polypeptide expression from at least age-matched fit individual. In an embodiment, the kit can also comprise a level of lamin precursor polypeptide expression from at least one age-matched fit individual. In still another embodiment, the kit can comprise a ratio value between the level of lamin A/C polypeptide expression and the level of lamin precursor polypeptide expression from at least one age-matched fit individual.

The kit can also comprise means for collecting a cell such as an instrument capable of retrieving a cell from a mucosa (for example the buccal mucosa), preferably in a non-invasive manner. Such means include, but are not limited to, a buccal swab or a brush.

The control value can also be provided in various ways. It can be provided as a single value that is aged-matched to the individual being tested. It can also be provided as a range (also referred to as a datastructure) that is provided as a function of age. These ranges of values as a function of age can be provided on a tangible medium (such as a print out or a computer-readable medium). Alternatively, the kit does not provide the datastructure itself but provide access to the datastructure. For example, the datastructure can be accessed via a computer or a portable device through a web-site or an application. Alternatively or in combination, the control value can be provided as a normogram or as access to a normogram.

As indicated above, the kit can comprise means for specifically detecting (and ultimately quantifying) the test level of lamin A/C polypeptide expression and/or the level of lamin precursor polypeptide expression. Such means include, but are not limited to an antibody or an antigen-binding fragment thereof recognizing the lamin A/C polypeptide and/or the lamin precursor polypeptide. Preferably, the antibodies are specific to their target polypeptide (e.g. the anti-lamin A/C antibody specifically recognizes the lamin A/C polypeptide and does not recognize the lamin precursor polypeptide and vice versa). In an embodiment, the antibody or a fragment thereof specific to the lamin precursor polypeptide recognizes the amino acid fragment present in a lamin precursor but absent in the mature lamin A/C polypeptide (e.g. for example the fragment cleaved off by the enzymatic activity of ZMPSTE24). The antibodies described herein can be polyclonal or monoclonal.

In an embodiment of the disclosure, the primary antibody recognizes an epitope present on mature lamin A (for example TRSYLLGNSSPRTQSPQNC). In another embodiment, the primary antibody recognizes an epitope present on prelamin A (for example TRSYLLGNSSPRT). In a further embodiment, the primary antibody recognizes an epitope present on progerin (for example GAQSPQNC).

Naturally occurring immunoglobulins have a common core structure in which two identical light chains (about 24 kD) and two identical heavy chains (about 55 or 70 kD) form a tetramer. The amino-terminal portion of each chain is known as the variable (V) region and can be distinguished from the more conserved constant (C) regions of the remainder of each chain. Within the variable region of the light chain is a C-terminal portion known as the J region. Within the variable region of the heavy chain, there is a D region in addition to the J region. Most of the amino acid sequence variation in immunoglobulins is confined to three separate locations in the V regions known as hypervariable regions or complementarity determining regions (CDRs) which are directly involved in antigen binding. Proceeding from the amino-terminus, these regions are designated CDR1, CDR2 and CDR3, respectively. The CDRs are held in place by more conserved framework regions (FRs). Proceeding from the amino-terminus, these regions are designated FR1, FR2, FR3, and FR4, respectively.

As used herein, the term antibody also includes antibody derivatives. Antibody derivatives include, but are not limited to, humanized antibodies. As used herein, the term "humanized antibody" refers to an immunoglobulin that comprises both a region derived from a human antibody or immunoglobulin and a region derived from a non-human antibody or immunoglobulin. The action of humanizing an antibody consists in substituting a portion of a non-human antibody with a corresponding portion of a human antibody. For example, a humanized antibody as used herein could comprise a non-human region variable region (such as a region derived from a murine antibody) capable of specifically recognizing the lamin A/C and/or the lamin precursor polypeptide and a human constant region derived from a human antibody. In another example, the humanized immunoglobulin can comprise a heavy chain and a light chain, wherein the light chain comprises a complementarity determining region derived from an antibody of non-human origin which specifically bind to the lamin A/C and/or the lamin precursor polypeptide and a framework region derived from a light chain of human origin, and the heavy chain comprises a complementarity determining region derived from an antibody of non-human origin which specifically binds the lamin A/C and/or the lamin precursor polypeptide and a framework region derived from a heavy chain of human origin.

As used herein, the present disclosure also relates to fragments of the antibodies described herein. As used herein, a "fragment" of an antibody (*e.g*. a monoclonal antibody) is a portion of an antibody that is capable of specifically recognizing the same epitope as the full version of the antibody. In the present disclosure, antibody fragments are capable of specifically recognizing the lamin A/C and/or the lamin precursor polypeptide. Antibody fragments include, but are not limited to, the antibody light chain, single chain antibodies, Fv, Fab, Fab' and F(ab')₂ fragments. Such fragments can be produced by enzymatic cleavage or by recombinant techniques. For instance, papain or pepsin cleavage can be used to generate Fab or F(ab')₂ fragments, respectively. Antibodies can also be produced in a variety of truncated forms using antibody genes in which one or more stop codons have been introduced upstream of the natural stop site. For example, a chimeric gene encoding the heavy chain of an F(ab')₂ fragment can be designed to include DNA sequences encoding the CH1 domain and hinge region of the heavy chain. Antibody fragments can also be humanized. For example, a humanized light chain comprising a light chain CDR *(i.e.* one or more CDRs) of non-human origin and a human light chain framework region. In another example, a humanized immunoglobulin heavy chain can comprise a heavy chain CDR *(i.e.* one or more CDRs) of non-human origin and a human heavy chain framework region. The CDRs can be derived from a non-human immunoglobulin.

The polyclonal antibody composition obtained by this method can be used for other purposes. The polyclonal antibody composition can be used directly as it is generated by the method, or can be further processed prior to its use. For example, the polyclonal antibody composition can be further fragmented, humanized, linked to another agent, etc.

The antibody composition can be coupled (*i.e.* physically linked) to a detectable substance. Such coupling may be direct (*e.g*. the antibody specifically recognizing the lamin A/C and/or the lamin precursor polypeptide is directly labeled) or indirect, through the use of a secondary labeled antibody. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, phycoerythrin, Alexa™ Fluor dye; an example of a luminescent material includes luminol; examples of bioluminescent materials include luciferase, luciferin, and aequorin, and examples of suitable radioactive materials include ¹²⁵I, ¹³¹I, ³⁵S or ³H. Alternatively, the antibody composition can be coupled to a chemotherapeutic agent; a toxin (*e.g*. an enzymatically active toxin of bacterial, fungal, plant, or animal origin, or fragments thereof); a radioactive isotope *(i.e.* a radioconjugate). Exemplary toxins include diphtheria A chain, nonbinding active fragments of diphtheria toxin, exotoxin A chain (from *Pseudomonas aeruginosa*)*,* ricin A chain, abrin A chain, modeccin A chain, alpha-sarcin, *Aleurites fordii* proteins, dianthin proteins, *Phytolaca americana* proteins (*e.g*. PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

When antibodies are provided for detecting the polypeptides, the kit can also comprise secondary antibodies for detecting the binding of the primary antibody to its cognate polypeptide target. Such secondary antibodies can be labeled for ease of detection. Alternatively or optionally, the kit can also comprise a solid support for at least one of the primary and/or secondary antibodies.

In another embodiment of the disclosure, the kit can also provide non-antibody means for detecting and quantifying the lamin A/C and/or lamin precursor. For example, if a mass spectrometry or a capillary electrophoresis technique is used to detect the polypeptides, the information necessary to detect specifically the polypeptides from a mass spectra or a electropherogram can be provided as means to detect the polypeptides.

The kit can also comprise additional components such as, for example, a container for collecting the cells collected with the means. The container may be provided with a solution for storing the cells (preferably a sterile solution).

The kit can also comprise instructions for collecting the cells. Such instructions can provide indications on where to collect the cells (the buccal mucosa for example), on how to collect the cells (by scrapping at least five times at a first location inside the screened individual's cheek) and on how to store the cells prior to the quantification step. Such instructions can be provided on a conventional support (such as paper), a computer-readable medium, as an hyperlink, etc.

The present invention will be more readily understood by referring to the following examples which are given to illustrate the invention rather than to limit its scope.

### EXAMPLE I - MATERIAL AND METHODS

*Human Buccal Cell Collection.* The subject's mouth is first rinsed with saline. The subject's inner cheek is gently scrapped with a sterile Cytobrush™ (Cooper Surgical, CT) following this pattern: five up and downs, rotate brush ≈120°, five up and downs, rotate until full rotation completed (≈20 seconds). The brush is then plunged into a 1.5 mL Eppendorf™ tube containing 1 mL of buccal cell (BC) buffer (PBS 1X, FBS 2%). This procedure is repeated two times for each cheek (*e.g*. front right cheek, back right cheek) and once for both upper and lower inner lips for a total of six tubes. The brush's handle is then cut and discarded and the tube lids are closed. The recuperated cells are stored at 4°C. Using this procedure, between 100 000 and 600 000 cells/mL were obtained. The cells collected are mostly epithelial buccal cells. No erythrocytes or lymphocytes were collected (data not shown).

*Preparation of Buccal Cells.* The tubes containing the stored cells are submitted to a vortex for 10 seconds at 1 500 rpm to detach the cells from the brush. The brush is then removed from the tubes using tweezers. The tubes are then centrifuged at 1500 *g* at of 4°C for 10 seconds to pellet the cells. The supernatant is removed. Then, 100 µL Trypsine-EDTA (previously heated to 37°C) is added to the pellet and incubated for 2 minutes. The pellet is resuspended by vortexing 5 seconds at 1 500 rpm. The pellet is re-centrifuged and washed twice using the BC buffer.

*Fixation of Buccal Cells.* When cells are stained immediately after their fixation, 250 µL of a Fix&Perm solution® (eBiosciences) is added to the cells which are then resuspended by vortexing 5 s at 1500 rpm. The cells are then incubated on a nutator mixer at 4°C for 20 min. The cells are washed twice in Perm/Wash@ buffer (eBiosciences). The cells are not further centrifuged after the second wash. On the other hand, when the staining of cells is deferred after their fixation, 100 µL of a 4% paraformaldehyde solution is added to the cells and the cells are resuspend by vortexing 5 seconds at 1500 rpm. The cells are then incubated on a nutator mixer at 4°C for 20 minutes. The cells are further washed cells two times in BC buffer. The cells are resuspended in BC buffer and store at 4°C for up to 48 hours. In either of the two fixation protocols, before starting the staining step, the cells are centrifuged 10 seconds at 1500 g at 4°C, the BC buffer is removed, and the cells are resuspended cells in 1 mL of Perm/Wash@ buffer for at least 15 minutes. The cells are filtered through a 50 µm nylon mesh disposable filter to remove remaining cell aggregates.

*Immunostaining.* The fixed cells are counted and their concentration is adjusted to 200 000 ± 25000 cells/mL. The cells are centrifuged and the supernatant is removed. Then, the antibodies are added. For the Lamin A/C isotype control, 1 µL of Lamin A/C isotype antibody (normal mouse IgG₂b (Santa Cruz, sc-3879)), 1 µL of anti-Lamin A antibody (Sigma #SAB4200420) or 1 µL of Prelamin A primary antibody are added to one tube (primary antibody final concentrations of 1:100). For the prelamin A isotype control, 1 µL of Prelamin A isotype antibody (normal goat IgG (Santa Cruz, sc-3887)) and 1 µL of Lamin A/C primary antibody are added to 1 tube (final concentrations of 1:100). For all other tubes, 1 µL of mouse monoclonal IgG₂b (Santa Cruz, sc-7292)), 1 µL of mouse IgG1 isotype control (Sigma # M5284) or 1 µL of goat polyclonal IgG (Santa Cruz, sc-6214)) are added (final concentrations of 1:100). The cells are resuspended and incubated 1 hour at 4°C on a nutator mixer. The cells are washed 2 times in Perm/Wash@ buffer. Then, 10 µL of a 1:545 dilution in Perm/Wash@ buffer of Prelamin A secondary antibody (Alexa Fluor 555 rabbit anti-goat IgG (Invitrogen, A21431)) is added (final concentration of 1:6000). The cells are resuspended and incubated 30 min on a nutator mixer at 4°C in the dark. The cells are washed twice in Perm/Wash@ buffer. Then, 10 µL of a 1:1091 dilution in Perm/Wash@ buffer of Lamin A/C secondary antibody (Alexa Fluor 647 goat anti-mouse IgG F(ab')₂) is added (final concentration of 1:12,000). The cells are resuspended and incubated 30 minutes on a nutator mixer at 4°C in the dark. The cells are washed twice in Perm/Wash@ buffer. Then, 100 µL Perm/Wash@ buffer is added to the pellet and mixed with the cells.

*Flow Cytometry.* The analysis were conducted on a BD LSFortessa™ and the voltages used for FSC, SSC, APC, PE were 93, 132, 392 and 314 respectively. A cell population was observed on a FSC-A *vs*. SSC-A dot plot. Gate 1 (G1) was placed to include 50-60% of the population closest to the center of mass (debris and multiplets). The gated cells (G1) were observed on a FSC-A vs. FSC-H dot plot. Gate 2 was placed to exclude G1 cells that are out of linearity (doublets). The gated cells (G2) were observed on an APC-A *vs.* PE-A dot plot. The Prelamin A isotype control samples were observed and gate 3 (G3) was placed on top of the G2 gated cells population to include -1% of them. G3 will discriminate between Prelamin A positive/negative cells in other samples. The Lamin A/C isotype control sample were observed and gate 4 (G4) was placed on the right of the G2 gated cells population to include -1% of them. G4 will discriminate between Lamin A/C positive/negative cells in other samples. The samples are then run until 30 000 G2 gated events are obtained. Alternatively, a total of 5000 cells re analyzed. The percentage of positive cells for lamin A/C and prelamin A for each sample as well as APC and PE mean fluorescence are then recorded.

### EXAMPLE II - DETECTION OF LAMIN A/C EXPRESSION IN BLOOD

Quantification of lamin A/C expression is desirable but was previously performed in samples acquired from invasive tissue biopsies. Although preliminary reports suggest that lamin A/C is not present in blood cells, it was prospectively verified whether lamin A/C could be quantified in nuclear extracts from isolated leukocytes.

Blood samples were obtained for 40 adult patients from the SMBD-Jewish General Hospital core laboratory: N=10 aged <30 years, N=10 aged 30-49, N=10 aged 50-69, and N=10 aged >=70. Patients with known hematologic, oncologic, or rheumatologic diseases were excluded (N=2), as were patients were significant laboratory abnormalities on those sampled (N=4). Blood samples were processed within 24 hours of phlebotomy. Leukocytes were isolated using standard Ficoll cell separation. Nuclear proteins were extracted from the isolated leukocytes and re-suspended in SDS electrophoresis buffer. Nuclear proteins were separated on SDS polyacrylamide gels and electro-transferred to polyvinyl membranes for Western Blot analysis. After being blocked with PBS, membranes were incubated overnight at 4°Celsius using two monoclonal antibodies, one directed against lamin A/C and another against lamin B1. Specific staining was revealed with horseradish peroxidase conjugated IgG followed by enhanced chemi-luminescence. The lamin A/C signals were quantified by densitometry and normalized according to lamin B1 signals. Lamin B1 signals were appropriately high in all samples, whereas lamin A/C signals were undetectable in all samples (data not shown). Subsequent experiments with a different lamin A/C antibody, and also with freshly collected blood samples confirmed the absence of lamin A/C in leukocytes nuclear extracts (data not shown). Lamin A/C was not found in detectable quantities in blood samples of human subjects of various age groups.

### EXAMPLE III - COMPARISON BETWEEN TELOMERE LENGTH, AORTIC LAMIN A/C EXPRESSION AND FRAILTY

A sub-study from clinical study of frailty in patients undergoing cardiac surgery (Afilalo J. *et al.,* 2010) was performed. Aortic and blood samples from 36 patients were obtained. The telomere length (RT-PCR), lamin A/C protein expression by immune-histochemistry and lamin A/C mRNA level relative to GADPH mRNA level by qPCR were determined from aortic sample. The telomere length was determined from the blood samples as well. Subjects were characterized as frail or non-frail based on the test elaborated in (Afilalo J. et al., J Am Coll Cardiol. 2010 Nov 9;56(20):1668-76).

In cardiac biopsies, lamin A/C protein expression, but not telomere length, is associated with frailty. More specifically, as shown in Figure 1A, the telomere length is not correlated with frailty, since telomere length is similar between frail and non-frail patients. However, as shown in Figures 1B , lamin A/C protein expression shows a correlation to frailty, e.g. a decreased level in lamin A/C expression is observed in patients characterized as frail.

In cardiac biopsies, lamin A/C protein expression, but not lamin A/C mRNA expression, is associated with frailty. More specifically, as shown in Figure 2A, the lamin A/C mRNA expression is not correlated with frailty, since lamin A/C mRNA expression is similar between frail and non-frail patients. However, as shown in Figure 2B, lamin A/C protein expression shows a correlation to frailty, *e.g*. a decreased level in lamin A/C expression is observed in patients characterized as frail.

Still in aortic biopsies, mature lamin A expression is decreased in frail patients (Figure 4A). In addition, progerin protein expression is increased in frail patients with respect to non-frail patients (Figure 4B). Further, the lamin A/C to progerin ratio is slightly increased in frail patients when compared to non-frail patients (Figure 4C).

In aortic biopsies, mature lamin A expression is decreased patients showing post-operative mortality or major comorbidity (Figure 6A). However, progerin protein expression as well as the ratio between lamin A/C and progerin are relatively similar between patients showing no incidence of post-operative mortality or major comorbidity and patients showing post-operative mortality or major comorbidity (Figures 6B and 6C).

In aortic paraformaldehyde-fixed biopsies, mature lamin A expression is increased in a fit patient (Figure 7A) when compared to a frail patient (Figure 7B). However, progerin expression is decreased in a fit patient (Figure 7C) when compared to a frail patient (Figure 7D).

### EXAMPLE IV- BUCCAL LAMIN A/C PROTEIN EXPRESSION AND FRAILTY

Lamin A/C's protein expression was determined in elderly patients undergoing cardiac surgery. A total of 12 patients were examined. Aortic and buccal samples were obtained from these patients (according to the methodology provided in Example I). Buccal lamin A/C's protein expression was determined according to the protocol provided in Example I. The time to walk five meters as well as the grip strength was assessed in these patients (as described in Afilalo J. *et al.* 2010).

Lamin A/C protein expression is inversely correlated to gait speed and positively correlated with grip strength. More specifically, as shown in Figure 3A, buccal lamin A/C protein expression is inversely correlated with the time to walk five meters, *e.g*. the lower the lamin expression, the longer it took the patient to walk five meters. As shown in Figure 3B, buccal lamin A/C protein expression is correlated with the grip strength, *e.g*. the lower the lamin expression, the lower the grip strength. Taken together, these results indicated that there is a correlation between buccal lamin A/C's protein expression and frailty (as indicated by the time to walk five meters and the grip strength).

It was also possible to perform flow cytopmetry to detect mature lamin-positive cells in buccal swabs (Figure 5).

Mature lamin A expression levels (as measured by flow cytometry) was shown to be increased in the buccal cells of fit patients when compared to frail patients (Figure 8A). Progerin expression levels (as measured by flow cytometry) was shown to be decreased in the buccal cells of fit patients when compared to frail patients (Figure 8B). The ratio between mature lamin A and progerin was increased in the buccal cells of fit patients when compared to frail patients (Figure 8C).

### REFERENCE

Afilalo J, Eisenberg MJ, Morin JF, Bergman H, Monette J, Noiseux N, Perrault LP, Alexander KP, Langlois Y, Dendukuri N, Chamoun P, Kasparian G, Robichaud S, Gharacholou SM, Boivin JF. Gait speed as an incremental predictor of mortality and major morbidity in elderly patients undergoing cardiac surgery. J Am Coll Cardiol. 2010 Nov 9;56(20):1668-76.

## Claims

1. A method of determining, in a screened individual, a risk of being frail, said method comprising:
(a) receiving a first test level of expression of at least one LMNA gene product in epithelial cells of a biological sample from the screened individual, the biological sample comprising the at least one LMNA gene product;
(b) comparing the first test of level expression of the at least one LMNA gene product to a first control level of expression of the at least one LMNA gene product to determine if the first test level is lower than, equal to or greater than the first control, wherein the first control level is the level of expression of the at least one LMNA gene product from an age-matched fit control individual; and
(c) characterizing the screened individual based on the comparison;
wherein the at least one LMNA gene product is selected from the group consisting of a mature lamin A-polypeptide and a ratio of the mature lamin A polypeptide to a lamin precursor polypeptide;
wherein step (c) further comprises characterizing the individual as (i) being more susceptible to being frail than the control individual if the first test level of is lower than the first control level, (ii) being as susceptible to being frail as the control individual if the first test level is equal to the first control level or (iii) being less susceptible to being frail than the control individual if the first test level is higher than the control level;
wherein the screened individual does not bear a genetic anomaly of a LMNA gene associated with progeria or a laminopathy.

2. The method of claim 1, wherein the lamin precursor polypeptide is selected from the group consisting of progerin, prelamin A and a combination thereof.

3. The method of any one of claims 1 to 2 wherein the first control level is a range of values of the level of expression of the at least one LMNA gene product obtained from age-matched control fit individuals.

4. The method of any one of claims 1 to 3 wherein the biological sample has been obtained from a mucosa.

5. The method of any one of claims 1 to 4 where the biological sample has been obtained from a buccal mucosa.

6. The method of any one of claims 1 to 5 wherein the screened individual is a human.

7. The method of any one of claims 1 to 6 wherein the first test level of expression of the at least one LMNA gene product is determined by flow cytometry.

8. The method of claim 4 wherein the mucosa is a lining mucosa.

## Patentansprüche

1. Verfahren zur Bestimmung eines Risikos für Gebrechlichkeit in einem untersuchten Individuum, wobei das Verfahren umfasst:
(a) Erhalten eines ersten Test-Expressionsspiegels mindestens eines LMNA-Genprodukts in Epithelzellen einer biologischen Probe des untersuchten Individuums, wobei die biologische Probe das mindestens eine LMNA-Genprodukt umfasst;
(b) Vergleichen des ersten Test-Expressionsspiegels des mindestens einen LMNA-Genprodukts mit einem ersten Kontroll-Expressionsspiegel des mindestens einen LMNA-Genprodukts, um zu bestimmen, ob der erste Testspiegel niedriger als, gleich oder höher als die erste Kontrolle ist, worin der erste Kontrollspiegel ein Expressionsspiegel des mindestens einen LMNA-Genprodukts von einem gesunden, altersangepassten Kontrollindividuum ist; und
(c) Charakterisieren der untersuchten Person auf Basis des Vergleichs;
worin das mindestens eine LMNA-Genprodukt ausgewählt ist aus der Gruppe bestehend aus maturem Lamin-A-Polypeptid und einem Verhältnis des maturen Lamin-A-Polypeptids zu einem Lamin-Vorläufer-Polypeptid;
worin Schritt (c) weiter umfasst das Charakterisieren des Individuums als (i) gefährdeter für Gebrechlichkeit als das Kontrollindividuum, wenn der erste Testspiegel niedriger ist als der erste Kontrollspiegel, (ii) gleich gefährdet für Gebrechlichkeit wie das Kontrollindividuum, wenn der erste Testspiegel gleich zu dem ersten Kontrollspiegel ist oder (iii) weniger gefährdet für Gebrechlichkeit als das Kontrollindividuum, wenn der erste Testspiegel höher ist als der Kontrollspiegel;
wobei das untersuchte Individuum keine genetische Anomalität eines LMNA-Gens, welche mit Progerie oder Laminopathie assoziiert ist, aufweist.

2. Verfahren nach Anspruch 1, worin das Lamin-Vorläufer-Peptid ausgewählt ist aus der Gruppe bestehend aus Progerin, Prelamin-A und einer Kombination hiervon.

3. Verfahren nach einem der Ansprüche 1 bis 2, worin der erste Kontrollspiegel ein Bereich von Werten des Expressionsspiegels des mindestens einen LMNA-Genprodukts ist, welche von gesunden altersangepassten Personen erhalten wurden.

4. Verfahren nach einem der Ansprüche 1 bis 3, worin die biologische Probe von einer Schleimhaut erhalten wurde.

5. Verfahren nach einem der Ansprüche 1 bis 4, worin die biologische Probe von einer Wangenschleimhaut erhalten wurde.

6. Verfahren nach einem der Ansprüche 1 bis 5, worin das untersuchte Individuum ein Mensch ist.

7. Verfahren nach einem der Ansprüche 1 bis 6, worin der erste Test-Expressionsspiegels des mindestens einen LMNA-Genprodukts durch Durchflusszytometrie bestimmt wird.

8. Verfahren nach Anspruch 4, worin die Schleimhaut eine auskleidende Schleimhaut ist.

## Revendications

1. Procédé de détermination, chez un individu examiné, d'un risque de fragilité, ledit procédé comprenant :
(a) la réception d'un premier niveau d'expression test d'au moins un produit de gène LMNA dans des cellules épithéliales d'un échantillon biologique issu de l'individu examiné, l'échantillon biologique comprenant l'au moins un produit de gène LMNA ;
(b) la comparaison du premier niveau d'expression test de l'au moins un produit de gène LMNA à un premier niveau d'expression témoin de l'au moins un produit de gène LMNA pour déterminer si le premier niveau test est inférieur, égal ou supérieur au premier niveau témoin, dans lequel le premier niveau témoin est le niveau d'expression de l'au moins un produit de gène LMNA d'un individu témoin adapté, apparié en fonction de l'âge ; et
(c) caractérisant l'individu examiné sur la base de la comparaison ;
dans lequel l'au moins un produit de gène LMNA est choisi dans le groupe consistant en un polypeptide de lamine A mature et un rapport du polypeptide de lamine A mature à un polypeptide précurseur de lamine ;
dans lequel l'étape (c) comprend en outre la caractérisation de l'individu comme (i) étant plus sensible à une fragilité que l'individu témoin si le premier niveau de test est inférieur au premier niveau témoin, comme (ii) étant sensible à une fragilité comme l'individu témoin si le premier niveau de test est égal au premier niveau témoin ou comme (iii) étant moins sensible à une fragilité que l'individu témoin si le premier niveau de test est supérieur au niveau témoin ;
dans lequel l'individu examiné ne porte pas d'anomalie génétique d'un gène LMNA associé à la progéria ou à une laminopathie.

2. Procédé selon la revendication 1,
dans lequel le polypeptide précurseur de la lamine est choisi dans le groupe consistant en progérine, prélamine A et une combinaison de celles-ci.

3. Procédé selon l'une quelconque des revendications 1 et 2,
dans lequel le premier niveau témoin est une plage de valeurs du niveau d'expression de l'au moins un produit de gène LMNA obtenu à partir d'individus témoins adaptés, appariés en fonction de l'âge.

4. Procédé selon l'une quelconque des revendications 1 à 3,
dans lequel l'échantillon biologique a été obtenu à partir d'une muqueuse.

5. Procédé selon l'une quelconque des revendications 1 à 4,
où l'échantillon biologique a été obtenu à partir d'une muqueuse buccale.

6. Procédé selon l'une quelconque des revendications 1 à 5,
dans lequel l'individu examiné est un être humain.

7. Procédé selon l'une quelconque des revendications 1 à 6,
dans lequel le premier niveau d'expression test de l'au moins un produit de gène LMNA est déterminé par cytométrie de flux.

8. Procédé selon la revendication 4, dans lequel la muqueuse est une muqueuse de revêtement.
